(19) **Europäisches Patentamt**

**European Patent Office**

**Office européen des brevets**

(11) **EP 0 512 855 B1**

(12) **EUROPEAN PATENT SPECIFICATION**

(45) Date of publication and mention
of the grant of the patent:
**01.08.2001 Bulletin 2001/31**

(51) Int Cl.[7]: **A61L 15/28**, A61L 15/58

(21) Application number: **92304163.6**

(22) Date of filing: **08.05.1992**

(54) **Absorbent wound filler**

Absorbierendes Wundenausfüllmittel

Matériau absorbant pour remplir les plaies

(84) Designated Contracting States:
**AT BE CH DE DK ES FR GB GR IT LI LU MC NL PT SE**

(30) Priority: **09.05.1991 US 697544**

(43) Date of publication of application:
**11.11.1992 Bulletin 1992/46**

(73) Proprietor: **E.R. SQUIBB & SONS, INC.**
**Princeton, New Jersey 08543-4000 (US)**

(72) Inventors:
• **Cilento,Rodolfo D.**
**New Jersey (US)**
• **Bolton,Laura Lee**
**New Jersey (US)**
• **Pirone,Louis A.**
**Pennsylvania (US)**

(74) Representative: **Nachshen, Neil Jacob et al**
**D Young & Co**
**21 New Fetter Lane**
**London EC4A 1DA (GB)**

(56) References cited:
**EP-A- 0 092 999**          **EP-A- 0 130 061**
**EP-A- 0 302 536**          **EP-A- 0 380 253**
**WO-A-90/01954**          **WO-A-90/10465**
**FR-A- 2 392 076**          **GB-A- 2 089 351**

Remarks:
The file contains technical information submitted after the application was filed and not included in this specification

**Description**

1. Field of the Invention

This invention relates to improved wound fillers. More particularly, this invention provides wound fillers having high moisture absorption capacity.

2. Description of the Prior Art

It is well known that the removal of wound exudates is important to the process of healing of wounds. Commonly used wound dressings utilize gauze, foams, sponges, cotton wads or other fibrous materials. Gauze and other fibrous materials absorb fluids by capillary action. However, gauze and other fibrous materials have the disadvantage in that when new tissue is formed, in the process of healing, it engulfs the fibers of these materials and it is torn when the material is removed causing wound injury on removal.

Various other materials have been used, such as gels, hydrogels, granules and pastes to remove exudates from wounds. These materials have the disadvantage of being difficult to remove from the wound after hydration.

U.S. Patent No. 4,551,490 describes an adhesive composition useful with ostomy and incontinent appliances and which has also been used in adhesive bandages. The composition consists of a homogeneous mixture of polyisobutylene, styrene radial or block-type copolymer, mineral oil, soluble hydrocolloid gum, water swellable cohesive strengthening agent and a tackifier. This composition has limited exudate absorption capacity and requires a tackifier for its intended use as an adhesive composition. WO-A-90/10465 describes an adhesive dressing for use with highly exuding wounds comprising a backing layer of a continuous polymeric moisture vapour transmitting film having a pressure sensitive adhesive on one side thereof in which the adhesive comprises a polyisobutylene in admixture with for example Na and Ca alginates.

**SUMMARY OF THE INVENTION**

The present invention provides wound filler having high exudate absorbing capacity. Generally the wound filler comprises from 25% to 75% by weight of a polymeric matrix and 25% to 75% by weight of absorbing powders. The polymeric matrix generally comprises from 15% to 75% by weight of one or more styrene radial or block type copolymers, from 5% to 40% by weight of one or more polyisobutylenes and from 5% to 40% of mineral oil. The absorbing powders generally contain from 10% to 100% by weight of sodium calcium alginates, from 0% to 80% by weight of cross-linked sodium carboxymethylcellulose from 0% to 80% by weight of absorbent polyacrylates and from 0% to 20% by weight of water soluble hydrocolloids.

The preferred wound filler of this invention can absorb 500% to 1000% its original weight, can be removed from the wound in one piece and does not cause wound injury on removal. The wound filler keeps the wound bed moist and produces in a wound an environment suitable for healing. The filler absorbs exudate without desiccating or dehydrating the wound bed, freshly generated tissue does not grow into it causing injury on removal. The absorbent wound filler of this invention is particularly useful for chronic heavily exudating wounds with large cavities.

DETAILED DESCRIPTION OF PREFERRED EMBODIMENTS

The absorbent wound filler of the present invention contains a polymeric matrix and absorbing powers dispersed within the matrix.

The wound filler contains from 25% to 75% by weight of polymeric matrix and 25% to 75% by weight of absorbing powders. Preferably, the wound filler contains 35% to 50% matrix and 50% to 65% absorbing powders.

The polymeric matrix is the structural component of the wound filler of this invention. It is sponge-like or a network of polymeric stretchable fibers or lamellas, within which the absorbing powders are entrapped. The polymeric matrix is a stretchable, elastic, sponge-like network of long chain molecules which form a mat-like structure. Embedded within this three-dimensional network are absorbing powders, powders capable of hydrating and swelling when exposed to exudate. The polymeric stretchable network allows the hydrating powders to swell and retains the powders in an integral structure but does not disintegrate under the forces of expansion. In addition, the polymeric matrix retains its physical properties when irradiated.

The polymeric matrix contains from 15% to 75% by weight of styrene radial or block type copolymers and from 5% to 40% by weight of mineral oil and from 5% to 40% by weight of polyisobutylene. The preferred polymeric matrix composition contains 40% to 60% styrene radial or block type copolymers and 20% to 30% mineral oil and 20% to 30% polyisobutylenes.

The styrene radial or block copolymer component of the wound filler provides structure and elasticity. The material permits swelling of the absorbent powders and does not break down when exposed to gamma radiation. These materials are described in U.S. Patent No. 4,551,490. Particularly suitable styrene copolymers include styrene-butadiene-styrene (S-B-S) and styrene-isoprene-styrene (S-I-S) block type copolymers both of which are commercially available, for example, from Shell Chemical Co. under the trademark Kraton™, as Kraton 1100™, 1101™, 1102™, 1107™, etc. The most preferred material is the styrene-isoprene-stryrene copolymer Kraton 1107™. One or more styrene-isoprene-styrene (S-I-S) block type copolymers may be employed.

The polyisobutylene component of the wound filler helps to bind the absorbing powders in the styrene radial or block copolymers network. It is a very viscous semi-solid material. Suitable polyisobutylene materials are described in U.S. Patent No. 4,551,490. The preferred polyisobutylenes are more low molecular weight polyisobutylenes having a viscosity average molecular weight of from about 36,000 to about 58,000 (Florey). Such polyisobutylenes are commercially available under the trademark Vistanex™ from Exxon as grades LMMS™ and LMMH™. Preferably, polyisobutylene Vistanex LMMH™ is used in the wound filler of this invention. If desired, 25% to 75% of the polyisobutylene can be substituted with butyl rubber.

The mineral oil functions as a plasticizer for the styrene radial or block copolymer component. It is also functions to increase the stretchability of the wound filler matrix.

The absorbing powders of the wound filler of the present invention constitute 25% to 75% by weight of the composition. In the preferred compositions, the absorbing powders are present in 50% to 65%. The powders for use in this invention absorb at least 300% by weight of the wound filler and preferably 500%. The absorbing powders useful in the invention have large water absorbing capacity, i.e., 1000% to 4000% by weight and are capable of being irradiated without substantial loss of water absorbing capacity. Additionally, they must not be easily leached out of the matrix when in contact with water.

The absorbing powders contain from 10% to 100%, preferably from 10% to 30%, by weight of sodium-calcium alginates such as those available under the tradename KELSET™ from Kelco Co., or mixtures of sodium alginate and calcium alginate commercially available under the tradename SOBALG Na Alginate™ and SOBALG Ca Alginate™ and commercially available from Grinsted of Denmark or mixtures of Na Alginate and Ca Alginate available under the tradenames PROTANAL Na Alginate and PROTANAL Ca Alginate™ from Protan of Norway. Also Preferably, the absorbing powders contain 10% to 75% by weight of sodium-calcium alginates.

In addition to the alginates, the absorbing powders contain from 0% to 80% and preferably 0% to 60% by weight of cross-linked sodium carboxymethylcellulose such as that commercially available under the tradename AcDiSol™ from FMC and under the tradename AKUCELL SWX 177™ from Akzo Co. of Holland and 0% to 80% and preferably 0% to 60% of finely divided substantially water insoluble highly absorbent polyacrylates representative of the highly absorbent polyacrylates are starch-graft copolymer such as that described in U.S. Patent No. 3,661,815 and commercially available from Grain Processing Corp. under the tradename WATER LOCK A100™ [a starch-graft-poly (sodium acrylate-co-acrylamide)], salt of cross-linked polyacrylic acid/polyalcohol grafted copolymer commercially available under the tradename FAVOR SAB800™ from Stockhausen, Inc., Greensboro, North Carolina, polyacrylate available under the tradename SALSORB 84™ from Allied Colloids, Inc., Suffolk, Virgina, sodium polyacrylate available under the tradename WATER LOCK J500™ from Grain Processing Corporation, cross-linked acrylic polymer under the trade name ARIDALL 1078™ from American Colloid Company, Skokie, Illinois, and potassium polyacrylate under the trade name ARASORB 732™ and 810™ from Arakawa Chemical Industries, Ltd., Osaka, Japan. WATER LOCK A100™ is the preferred polyacrylate.

Suitable water soluble hydrocolloids include sodium carboxymethylcellulose, pectin, which is preferred, gelatin, guar gum, locust bean gum, gum karaya and mixtures thereof. The water soluble hydrocolloids are present in an amount of from 0% to 20% of the weight and preferably 5% to 15%.

The wound filler composition can, if desired, contain small amounts, i.e., less than 1%, of pharmacologically active ingredients. For example, an antibiotic or antimicrobial agent such as neomycin or penicillin, an antiseptic agent such as povidone iodine, an anti-inflammatory agent such as hydrocortisone or triamcinolone acetonides, or a skin protective agent such as zinc oxide can be included in the composition.

The wound filler of this invention is prepared by mixing and heating the styrene copolymers and mineral oil in a heavy duty high shear sigma blade mixer. The mixture is heated from about 125°C to about 150°C, preferably 150°C, and the mixing is continued until the mass is homogeneous. The mixture is then cooled to 125°C and polyisobutylene is added and the mixture is mixed until a soft plastic, lightly tacky mass is formed. After cooling to about 100°C, the absorbing powders are added and material is mixed to form a dough-like mass. The doughy mass is calendered through a double roll mill between two sheets of release paper at 100°C to 125°C, preferably, about 125°C. A flexible sheet or slab of any desired thickness (20, 40, 60, 80, 100 mils (2.54mm) depending on the gap set between the two rolls) is formed, 40 to 60 mils (1.02 to 1.52 mm) thickness is desired. The slabs are die cut to any desired size and shape, sealed in pouches and may be sterilized by means of gamma radiation.

EXAMPLE 1

Absorbent wound filler was prepared having the following composition:

| | Weight Percent Of Wound Filler |
|---|---|
| Polyisobutylene (Vistamex LMMH™) Styrene-isoprene-styrene copolymer (Kraton 1107™) | 10 20 |
| Mineral Oil | 10 |
| Pectin | 10 |
| Na Ca Alginates | 25 |
| Cross-linked sodium carboxymethylcellulose (Ac-Di-Sol™) | 25 $\overline{100}$ |

Kraton™ (60g) is mixed with mineral oil (30g) in a sigma blade mixer at 150°C. Polyisobutylene (30g) is added and mixed after cooling to 125°C for ten minutes. A soft plastic mass is formed. The mixture is cooled to 100°C and pectin (30g), Na Ca Alginates (75g) and cross-linked sodium carboxymethylcellulose (75g) are added and mixed at 100°C for 30 minutes until a homogeneous dough-like mass is obtained.

The doughy mass is calandered through a double roll mill between two sheets of release paper at 125°C. A flexible slab of 60 mils (1.52mm) is formed.

The slabs are die cut to 1" x 2", 2" x 2" and 2" x 3" and circular discs of 1", 2" and 3" diameter and sealed in pouches and gamma irradiated at 2.5 MR or 25KGy (1" = 2.54 cm, 2" = 5.08 cm and 3" = 7.62 cm).

The moisture absorption of the absorbent wound filler is determined according to the following procedure:

(1) Samples of 1" x 2" (2.54 cm x 5.08 cm) are weighed and placed into sealed cups full of saline solution at 40°C.

(2) Periodically the samples are removed from the solution, patted dry on a towel to remove superficial water, weighed, placed back into the cups.

(3) The weights are taken at 0.25, 0.5, 1.0, 2.0, 4.0, 8.0, 24.0 hours initially, and then daily for one week.

(4) The moisture absorption is expressed in percent of initial weight and it is calculated by the following formula:

$$\% \text{ Moist Absorption} = \frac{Wt - Wi}{Wi} \times 100$$

Wt = Weight at various times
Wi = Initial Weight

When the absorbent wound filler of this Example (irradiated and non-irradiated) is tested for moisture absorption according to the above procedure the following results are obtained:

| Water Absorption Capacity (% of Initial Weight) | | | | | | | | |
|---|---|---|---|---|---|---|---|---|
| Time (Hours) | 2 | 6 | 8 | 24 | 48 | 144 | 216 | 312 |
| Sample | | | | | | | | |
| Irradiated | 84 | 271 | 313 | 465 | 543 | 589 | 580 | 541 |
| Non-Irradiated | 67 | 255 | 315 | 564 | 656 | 716 | 716 | 702 |

EXAMPLE 2

Absorbent wound filler was prepared having the following composition:

|  | Weight Percent Of Wound Filler |
|---|---|
| MATRIX 45% |  |
| Polyisobutylene (Vistamex LMMI™) | 11.25 |
| Styrene-isoprene-styrene copolymer (Kraton 1107™) | 22.5 |
| Mineral Oil | 11.25 |
| POWDERS 55% |  |
| Pectin | 10 |
| Na Ca Alginates (Kelset) | 15 |
| Starch graft copolymer - (WATERLOCK A100™) | $\underline{30}$ <br> $\overline{100}$ |

Kraton™ (67.50g) is mixed with mineral oil (33.75g) in a sigma blade mixer at 150°C. Polyisobutylene (33.75g) is added and mixed after cooling to 125°C for ten minutes. A soft plastic mass is formed. The mixture is cooled to 100°C and pectin (30.00g), Na Ca Alginates (45.00g) and starch graft copolymer (polyacrylate and polyacrylamide) are added and mixed at 100°C for 30 minutes until a homogeneous dough-like mass is obtained.

The doughy mass is calandered through a double roll mill between two sheets of release paper at 125°C. A flexible slab of a desired thickness of 60 mils (1.52 mm) is formed.

The slabs are die cut to 1" x 2", 2" x 2" and 2" x 3" and circular discs of 1", 2" and 3" diameter and sealed in pouches and gamma irradiated at 2.5 MR or 25 KGy (1" = 2.54 cm, 2" = 5.08 cm and 3" = 7.62 cm).

When the absorbent wound filler of this Example (irradiated) is tested for moisture absorption according to the procedure described in Example 1 the following results are obtained:

| RATE OF ABSORPTION AVERAGE OF RESULTS ||
|---|---|
| Time Interval | % Weight Gain |
| 15 minutes | 92% |
| 30 minutes | 147% |
| 1 hour | 258% |
| 2 hours | 469% |
| 4 hours | 629% |
| 8 hours | 743% |
| 24 hours | 818% |
| 48 hours | 858% |
| 72 hours | 870% |
| 96 hours | 916% |
| 120 hours | 956% |

EXAMPLE 3

Absorbent wound filler was prepared having the following composition:

|  | Weight Percent Of Wound Filler |
|---|---|
| MATRIX 40% |  |
| Polyisobutylene (Vistamex LMMH™) | 10 |
| Styrene-isoprene-styrene copolymer (Kraton 1107™) | 20 |
| Mineral Oil | 10 |
| POWDERS 60% |  |
| Pectin | 10 |
| Na Ca Alginates (Kelset) | 20 |

(continued)

|  | Weight Percent Of Wound Filler |
|---|---|
| POWDERS 60% | |
| Cross-linked sodium carboxymethylcellulose (Ac-Di-Sol™ 15%, Akucell SW x 177™ 15%) | 30 <br> $\overline{100}$ |

Kraton™ (60.00g) is mixed with mineral oil (30.00g) in a sigma blade mixer at 150°C. Polyisobutylene (30.00g) is added and mixed after cooling to 125°C for ten minutes. A soft plastic mass is formed. The mixture is cooled to 100°C and pectin (30.00g), Na Ca Alginates (60.00g) and cross-linked sodium carboxymethylcellulose (90.00g) are added and mixed at 100°C for 30 minutes until a homogeneous dough-like mass is obtained.

The doughy mass is calandered through a double roll mill between two sheets of release paper at 125°C. A flexible slab of a desired thickness of 60 mils (1.52 mm) is formed.

The slabs are die cut to 1" x 2", 2" x 2" and 2" x 3" and circular discs of 1", 2" and 3" diameter and sealed in pouches and gamma irradiated at 2.5 MR or KGy (1" = 2.54 cm, 2" = 5.08 cm and 3" = 7.62 cm).

When the absorbent wound filler of this Example (irradiated) is tested for moisture absorption according to the procedure described in Example 1 the following results are obtained:

| RATE OF ABSORPTION AVERAGE OF RESULTS | |
|---|---|
| Time Interval | % Weight Gain |
| 15 minutes | 60% |
| 30 minutes | 88% |
| 1 hour | 142% |
| 2 hours | 244% |
| 4 hours | 327% |
| 8 hours | 393% |
| 24 hours | 466% |
| 48 hours | 502% |
| 72 hours | 534% |
| 96 hours | 542% |
| 120 hours | 545% |

EXAMPLES 4 - 10

Following the procedure described in Example 1 and using the formulations shown in Table I, seven absorbent wound fillers were prepared and wound fillers of Examples 5, 6, 7 and 8 evaluated for moisture absorption according to the procedure described in Example 1. The moisture absorption test results are set forth in Table II.

TABLE I

| | | WEIGHT % OF WOUND FILLER ABSORBING POWDERS | | | |
|---|---|---|---|---|---|
| | Polymeric Matrix | Pectin | Na Ca Alginates | Crosslinked Na CMC | Water Lock A100™ |
| EXAMPLE | | | | | |
| 4 | 40 (1) | | 60 | | |
| 5 | 40 (1) | | 10 | 50*** | |
| 6 | 40 (1) | 10 | 40 | | 10 |
| 7 | 40 (1) | 10 | 50 | | |
| 8 | 45 (2) | 10 | 22.5 | 22.5* | |

(2) Polymeric matrix is the same as that of Example 2

(2) Polymeric matrix is the same as that of Example 2

* AcDiSol™

*** A blend of equal parts of AcDiSol™ and Akucell x177™

TABLE I   (continued)

| | | WEIGHT % OF WOUND FILLER ABSORBING POWDERS | | | |
|---|---|---|---|---|---|
| | Polymeric Matrix | Pectin | Na Ca Alginates | Crosslinked Na CMC | Water Lock A100™ |
| 9 | 40(1) | 10 | 25 | 25** | |
| 10 | 40 (1) | 10 | 20 | 30*** | |

(1) Polymeric matrix is the same as that of Example 1

** Akucell x177™

*** A blend of equal parts of AcDiSol™ and Akucell x177™

**TABLE II**

Water Absorption Capacity
(% of Initial Weight)

| Time (Hours) | 2 | 4 | 6 | 8 | 24 | 48 | 120 | 144 | 216 | 312 |
|---|---|---|---|---|---|---|---|---|---|---|
| **EXAMPLE 5** | | | | | | | | | | |
| Irradiated | 138 | | 401 | 419 | 551 | 612 | | 704 | 658 | 539 |
| Non-Irradiated | 97 | | 287 | 300 | 363 | 386 | | 427 | 413 | 369 |
| **EXAMPLE 6** | | | | | | | | | | |
| Irradiated | | 453 | | 537 | 662 | 688 | 687 | 883 | | |
| Non-Irradiated | | 412 | | 644 | 854 | 911 | 1413 | 1572 | | |
| **EXAMPLE 7** | | | | | | | | | | |
| Irradiated | | 237 | | 412 | 516 | 564 | 616 | 621 | | |
| Non-Irradiated | | 352 | | 610 | 765 | 842 | 911 | 922 | | |
| **EXAMPLE 8** | | | | | | | | | | |
| Irradiated | 83 | | 258 | 297 | 429 | 493 | | 555 | 562 | 543 |
| Non-Irradiated | 94 | | 270 | 326 | 552 | 615 | | 672 | 644 | 610 |

EXAMPLE 11

The following results were obtained from wound studies conducted on pigs:

| % INCREASE IN WEIGHT OF WOUND FILLER | | | | |
|---|---|---|---|---|
| | DAY 1 | DAY 2 | DAY 4 | DAY 7 |
| Sample | | | | |
| EXAMPLE 2 | 468 | 426 | 449 | 285 |
| EXAMPLE 10 | 341 | 306 | 390 | 264 |

15 mm dia. x 1.75 mm thick discs of the wound filler of Examples 2 and 10 were placed in 25 mm dia. x 5 mm depth wounds and covered with a 4" x 4" (10.16 cm x 10.16 cm) dressing. Initially and at the end of days 1, 2 and 4 the wound filler was weighed and replaced with a fresh dry wound filler disc; at the end of day 7 the wound filler was weighed but not replaced. The percent increase of weight was calculated.

The wound fillers of the present invention absorb large quantities of wound exudate. In the process of absorbing fluids they swell and fill the wound cavity. They can absorb 500% to 1000% of their original weight and swell proportionally, i.e., they can swell 5 - 10 times their original volume. The wound fillers absorb by hydration and can be removed from the wound in one piece. They do not cause wound injury on removal and keep the wound bed moist and in an environment suited for healing without desiccating or dehydrating the wound bed. The wound fillers do not adhere to the wound bed and freshly generated tissue does not grow into the filler causing injury on removal. Their main application is directed to chronic heavily exudating wounds, with large cavities, where absorption and filling are indicated. The wound filler also finds use in treatment of ulcers, burns, pressure sores, etc.

The wound filler of any desired thickness is applied to the wound. Usually 1/5 to 1/4 of the wound cavity is filled with a small piece of wound filler and the wound sealed with an occlusive dressing. As exudate is generated it is absorbed by the wound filler which swells to fill the entire wound cavity. Usually the wound filler will not be required to be replaced for several days.

**Claims**

1.  An absorbent wound filler composition having an absorbence capacity of at least 300% based on initial weight of said wound filler which comprises:

    (a) from 25% to 75% by weight of a polymeric matrix wherein said matrix comprises

        (i) from 15% to 75% by weight of one or more styrene radial or block type copolymers;
        (ii) from 5% to 40% by weight of one or more polyisobutylenes; and
        (iii) from 5% to 40% by weight of mineral oil.

    (b) from 25% to 75% by weight of absorbing powders wherein said absorbing powders comprise

        (i) from 10% to 100% by weight of sodium calcium alginates;
        (ii) from 0% to 80% by weight of cross-linked sodium carboxymethylcellulose;
        (iii) from 0% to 80% by weight of absorbent polyacrylates; and
        (iv) from 0% to 20% by weight of water soluble hydrocolloids.

2.  The composition of Claim 1 wherein said composition comprises from 35% to 50% polymeric matrix and from 50% to 65% absorbing powders.

3.  The composition of Claim 1 or 2 wherein the absorbent polyacrylate is present and is a starch-graft poly (sodium acrylate-co-acrylate) copolymer.

4.  The composition of Claim 1, 2 or 3 wherein the water soluble hydrocolloid is present and is pectin.

5.  The composition of Claim 1, 2, 3 or 4 wherein said absorbing powders comprises from 10% to 30% by weight of Na Ca alginates.

6.  The composition of Claim 1 wherein said composition comprises:

(a) about 20% by weight of styrene-isoprene-styrene copolymer;
(b) about 10% by weight of polyisobutylene;
(c) about 10% by weight of mineral oil;
(d) about 25% by weight of Na Ca alginates;
(e) about 25% by weight of cross-linked sodium carboxymethylcellulose; and
(f) about 10% by weight of pectin.

7. The composition of Claim 1 wherein said composition comprises:

(a) about 22.5% by weight of styrene-isoprene-styrene copolymer;
(b) about 11.25% by weight of polyisobutylene;
(c) about 11.25% by weight of mineral oil;
(d) about 15% by weight of Na Ca alginates;
(e) about 30% by weight of starch-graft poly (sodium acrylate-co-acrylate); and
(f) about 10% by weight of pectin.

8. The composition of Claim 1 wherein said composition comprises:

(a) about 20% by weight of styrene-isoprene-styrene copolymer;
(b) about 10% by weight of polyisobutylene;
(c) about 10% by weight of mineral oil;
(d) about 20% by weight of Na Ca alginates;
(e) about 30% by weight of cross-linked sodium carboxymethylcellulose; and
(f) about 10% by weight of pectin.

9. The composition of Claim 1 wherein said composition comprises:

(a) about 20% by weight of styrene-isoprene-styrene copolymer;
(b) about 10% by weight of polyisobutylene;
(c) about 10% by weight of mineral oil;
(d) about 40% by weight of Na Ca alginates;
(e) about 10% by weight of starch-graft poly (sodium acrylate-co-acrylate); and
(f) about 10% by weight of pectin.

10. The composition of Claim 1 wherein said composition comprises:

(a) about 20% by weight of styrene-isoprene-styrene copolymer;
(b) about 10% by weight of polyisobutylene;
(c) about 10% by weight of mineral oil;
(d) about 50% by weight of Na Ca alginates; and
(e) about 10% by weight of pectin.

11. The composition of Claim 1 wherein said composition comprises:

(a) about 22.5% by weight of styrene-isoprene-styrene copolymer;
(b) about 11.25% by weight of polyisobutylene;
(c) about 11.25% by weight of mineral oil;
(d) about 22.5% by weight of Na Ca alginates;
(e) about 22.5% by weight of cross-linked sodium carboxymethylcellulose; and
(f) about 10% by weight of pectin.

12. Use of a composition of any preceding claim for the manufacture of a medicament for treatment of an exudating wound, in which the medicament fills the wound cavity and the wound and the medicament are covered with an occlusive dressing.

**Patentansprüche**

1. Absorbierende Wundfüllstoffzusammensetzung mit einer Absorptionskapazität von mindestens 300%, bezogen auf das Anfangsgewicht dieses Wundfüllstoffs, die umfaßt:

   (a) 25 Gew.-% bis 75 Gew.-% einer Polymermatrix, wobei diese Matrix umfaßt

   (i) 15 Gew.-% bis 75 Gew.-% eines oder mehrerer radialer oder blockartiger Styrol-Copolymere,
   (ii) 5 Gew.-% bis 40 Gew.-% eines oder mehrerer Polyisobutylene und
   (iii) 5 Gew.-% bis 40 Gew.-% Mineralöl;

   (b) 25 Gew.-% bis 75 Gew.-% absorbierende Pulver, wobei diese absorbierenden Pulver umfassen

   (i) 10 Gew.-% bis 100 Gew.-% Natriumcalciumalginate;
   (ii) 0 Gew.-% bis 80 Gew.-% vernetzte Natriumcarboxymethylcellulose;
   (iii) 0 Gew.-% bis 80 Gew.-% absorbierende Polyacrylate und
   (iv) 0 Gew.-% bis 20 Gew.-% wasserlösliche Hydrokolloide.

2. Zusammensetzung nach Anspruch 1, wobei diese Zusammensetzung 35% bis 50% Polymermatrix und 50% bis 65% absorbierende Pulver umfaßt.

3. Zusammensetzung nach Anspruch 1 oder 2, wobei das absorbierende Polyacrylat anwesend ist und ein Stärke-Pfropf-Poly(natriumacrylat-co-acrylat)copolymer ist.

4. Zusammensetzung nach Anspruch 1, 2 oder 3, wobei das wasserlösliche Hydrokolloid anwesend ist und Pektin ist.

5. Zusammensetzung nach Anspruch 1, 2, 3 oder 4, wobei diese absorbierenden Pulver 10 Gew.-% bis 30 Gew.-% Na-Ca-Alginate umfassen.

6. Zusammensetzung nach Anspruch 1, wobei diese Zusammensetzung:

   (a) etwa 20 Gew.-% Styrol-Isopren-Styrol-Copolymer;
   (b) etwa 10 Gew.-% Polyisobutylen;
   (c) etwa 10 Gew.-% Mineralöl;
   (d) etwa 25 Gew.-% Na-Ca-Alginate;
   (e) etwa 25 Gew.-% vernetzte Natriumcarboxymethylcellulose und
   (f) etwa 10 Gew.-% Pektin umfaßt.

7. Zusammensetzung nach Anspruch 1, wobei diese Zusammensetzung:

   (a) etwa 22,5 Gew.-% Styrol-Isopren-Styrol-Copolymer;
   (b) etwa 11,25 Gew.-% Polyisobutylen;
   (c) etwa 11,25 Gew.-% Mineralöl;
   (d) etwa 15 Gew.-% Na-Ca-Alginate;
   (e) etwa 30 Gew.-% Stärke-Pfropf-Poly(natriumacrylat-co-acrylat) und
   (f) etwa 10 Gew.-% Pektin umfaßt.

8. Zusammensetzung nach Anspruch 1, wobei diese Zusammensetzung:

   (a) etwa 20 Gew.-% Styrol-Isopren-Styrol-Copolymer;
   (b) etwa 10 Gew.-% Polyisobutylen;
   (c) etwa 10 Gew.-% Mineralöl;
   (d) etwa 20 Gew.-% Na-Ca-Alginate;
   (e) etwa 30 Gew.-% vernetzte Natriumcarboxymethylcellulose und
   (f) etwa 10 Gew.-% Pektin umfaßt.

9. Zusammensetzung nach Anspruch 1, wobei diese Zusammensetzung:

(a) etwa 20 Gew.-% Styrol-Isopren-Styrol-Copolymer;
(b) etwa 10 Gew.-% Polyisobutylen;
(c) etwa 10 Gew.-% Mineralöl;
(d) etwa 40 Gew.-% Na-Ca-Alginate;
(e) etwa 10 Gew.-% Stärke-Pfropf-Poly(natriumacrylat-co-acrylat) und
(f) etwa 10 Gew.-% Pektin umfaßt.

10. Zusammensetzung nach Anspruch 1, wobei diese Zusammensetzung:

(a) etwa 20 Gew.-% Styrol-Isopren-Styrol-Copolymer;
(b) etwa 10 Gew.-% Polyisobutylen;
(c) etwa 10 Gew.-% Mineralöl;
(d) etwa 50 Gew.-% Na-Ca-Alginate und
(e) etwa 10 Gew.-% Pektin umfaßt.

11. Zusammensetzung nach Anspruch 1, wobei diese Zusammensetzung:

(a) etwa 22,5 Gew.-% Styrol-Isopren-Styrol-Copolymer;
(b) etwa 11,25 Gew.-% Polyisobutylen;
(c) etwa 11,25 Gew.-% Mineralöl;
(d) etwa 22,5 Gew.-% Na-Ca-Alginate;
(e) etwa 22,5 Gew.-% vernetzte Natriumcarboxymethylcellulose und
(f) etwa 10 Gew.-% Pektin umfaßt.

12. Verwendung einer Zusammensetzung nach einem der vorstehenden Ansprüche zur Herstellung eines Medikamentes zur Behandlung einer exsudierenden Wunde, wobei das Medikament die Wundhöhle füllt und die Wunde und das Medikament mit einem Okklusionsverband bedeckt werden.

## Revendications

1. Composition absorbante de remplissage de plaies possédant une capacité d'absorption au moins égale à 300% par rapport au poids initial de ladite composition de remplissage, comprenant:

(a) de 25% à 75% en poids d'une matrice polymère, ladite matrice comprenant

(i) de 15% à 75% en poids d'un ou plusieurs copolymères du styrène, de type radial ou séquencé;
(ii) de 5% à 40% en poids d'un ou plusieurs polyisobutylènes; et
(iii) de 5% à 40% en poids d'huile minérale;

(b) de 25% à 75% en poids de poudres absorbantes, lesdites poudres absorbantes comprenant

(i) de 10% à 100% en poids d'alginates de sodium et de calcium;
(ii) de 0% à 80% en poids de carboxy-méthylcellulose sodique réticulée;
(iii) de 0% à 80% en poids de polyacrylates. absorbants; et
(iv) de 0% à 20% en poids d'hydrocolloïdes solubles dans l'eau.

2. Composition selon la revendication 1, comprenant de 35% à 50% de matrice polymère et de 50% à 65% de poudres absorbantes.

3. Composition selon la revendication 1 ou 2, comprenant le polyacrylate absorbant, lequel est un copolymère greffé de poly(acrylate-co-acrylate de sodium) et d'amidon.

4. Composition selon la revendication 1, 2 ou 3, comprenant l'hydrocolloïde soluble dans l'eau, lequel est la pectine.

5. Composition selon la revendication 1, 2, 3 ou 4, dans laquelle lesdites poudres absorbantes comprennent de 10% à 30% en poids d'alginates de sodium et de calcium.

**6.** Composition selon la revendication 1, comprenant:

(a) environ 20% en poids de copolymère styrène-isoprène-styrène;
(b) environ 10% en poids de polyisobutylène;
(c) environ 10% en poids d'huile minérale;
(d) environ 25% en poids d'alginates de sodium et de calcium;
(e) environ 25% en poids de carboxyméthyl-cellulose sodique réticulée; et
(f) environ 10% en poids de pectine.

**7.** Composition selon la revendication 1, comprenant:

(a) environ 22,5% en poids de copolymère styrène-isoprène-styrène;
(b) environ 11,25% en poids de polyisobutylène;
(c) environ 11,25% en poids d'huile minérale;
(d) environ 15% en poids d'alginates de sodium et de calcium;
(e) environ 30% en poids de copolymère greffé de poly(acrylate-co-acrylate de sodium) et d'amidon; et
(f) environ 10% en poids de pectine.

**8.** Composition selon la revendication 1, comprenant:

(a) environ 20% en poids de copolymère styrène-isoprène-styrène;
(b) environ 10% en poids de polyisobutylène;
(c) environ 10% en poids d'huile minérale;
(d) environ 20% en poids d'alginates de sodium et de calcium;
(e) environ 30% en poids de carboxyméthyl-cellulose sodique réticulée; et
(f) environ 10% en poids de pectine.

**9.** Composition selon la revendication 1, comprenant:

(a) environ 20% en poids de copolymère styrène-isoprène-styrène;
(b) environ 10% en poids de polyisobutylène;
(c) environ 10% en poids d'huile minérale;
(d) environ 40% en poids d'alginates de sodium et de calcium;
(e) environ 10% en poids de copolymère greffé de poly(acrylate-co-acrylate de sodium) et d'amidon; et
(f) environ 10% en poids de pectine.

**10.** Composition selon la revendication 1, comprenant:

(a) environ 20% en poids de copolymère styrène-isoprène-styrène;
(b) environ 10% en poids de polyisobutylène;
(c) environ 10% en poids d'huile minérale;
(d) environ 50% en poids d'alginates de sodium et de calcium; et
(e) environ 10% en poids de pectine.

**11.** Composition selon la revendication 1, comprenant:

(a) environ 22,5% en poids de copolymère styrène-isoprène-styrène;
(b) environ 11,25% en poids de polyisobutylène;
(c) environ 11,25% en poids d'huile minérale;
(d) environ 22,5% en poids d'alginates de sodium et de calcium; et
(e) environ 22,5% en poids de carboxyméthyl-cellulose sodique réticulée; et
(f) environ 10% en poids de pectine.

**12.** Utilisation d'une composition selon l'une quelconque des revendications précédentes pour la fabrication d'un médicament permettant le traitement d'une plaie suintante, le médicament remplissant la cavité de la plaie, et la plaie et le médicament étant recouverts d'un pansement occlusif.